# EUROPEAN PATENT APPLICATION

(11) **EP 4 407 630 A1**
(43) Date of publication of application: **31.07.2024**
(21) Application number: 24153570.7
(22) Date of filing: 24.01.2024
(51) Int. Cl.: G16H 30/40, A61B 5/026, A61B 8/08, G16H 50/20, G16H 50/30, G16H 50/50

(54) **ARTIFICIAL INTELLIGENCE-BASED STROKE RISK PREDICTION FROM CAROTID ARTERY IMAGING INFORMATION**

(30) Priority: 25.01.2023 US 202363481396 P; 25.05.2023 US 202318323566
(71) Applicant: Siemens Medical Solutions USA, Inc., Malvern, PA 19355 (US)
(72) Inventor: PASSERINI, Tiziano, Plainsboro, NJ, 08536 (US); EL SAYED, Retta, Atlanta, GA, 30324 (US)
(74) Representative: Horn Kleimann Waitzhofer Schmid-Dreyer Patent- und Rechtsanwälte PartG mbB

(57) **Abstract**

For predicting (140) stroke risk, an artificial intelligence (450) rapidly generates (140) flow information from input of geometric parameters of a carotid of a patient. An image processor (420) predicts (152) the stroke risk from the flow information. In one approach, the values of the geometric parameters of the carotid of the patient are perturbed based on uncertainty (130). The artificial intelligence (450) generates (140) candidate flow information for each perturbation. The candidate flow information sufficiently matching (150) a measurement of flow for the patient is used as the flow information for stroke risk prediction (152).

## Description

### RELATED APPLICATION

The present patent document claims the benefit of the filing date under 35 U.S.C. § 119(e) of Provisional U.S. Patent Application Serial No. 63/481,396, filed January 25, 2023, which is hereby incorporated by reference.

### FEDERALLY SPONSORED RESEARCH OR DEVELOPMENT

This invention was made with government support under NIH R01EB027774 awarded by NIH. The government has certain rights in the invention.

### BACKGROUND

The present embodiments relate to stroke risk prediction. Carotid arteries provide the main blood supply to the brain. Carotid artery disease results from a buildup of atherosclerotic plaque. Plaque can rupture, triggering thrombus formation in the carotid bifurcation, which formation can lead to ischemic stroke.

The geometry of the bifurcation is a good predictor of altered flow conditions and therefore of increased stroke risk. Carotid flow may be studied based on computational fluid dynamics (CFD). CFD uses patient-specific geometry and input flow and pressure boundary conditions. Patient-specific geometry requires imaging and a long preprocessing time for segmentation and meshing. This approach has many limitations due to the multiple required assumptions (e.g., rigid vascular walls), long processing time, and requirement of high computational power. Another method for quantifying blood flow is four-dimensional (4D) flow magnetic resonance imaging (MRI). 4D flow MRI provides a three-dimensional (3D) anatomy image and a 3D time-resolved velocity field, so may be used to assess both vascular geometry and different hemodynamics parameters. 4D flow MRI has a long (e.g., 10 minutes) clinical scan time, long image processing time, and limited anatomical image quality, which can be challenging for accurate vessel segmentation. Hemodynamics near the vessel wall derived from 4D flow MRI may be inaccurate due to the limited spatial resolution. Analysis of geometry and flow based on medical images of the carotid bifurcation is inefficient and requires complex workflows with significant processing time, leading to limited adoption in clinical workflows.

### SUMMARY

The invention is defined by the enclosed claims, wherein systems, methods, and non-transitory computer readable media with instructions are provided for predicting stroke risk with an artificial intelligence. The artificial intelligence rapidly generates flow information from input of geometric parameters of a carotid of a patient. An image processor predicts the stroke risk from the flow information. In one approach, the values of the geometric parameters of the carotid of the patient are perturbed based on uncertainty. The artificial intelligence generates candidate flow information for each perturbation. The candidate flow information sufficiently matching a measurement of flow for the patient is used as the flow information for stroke risk prediction.

In a first aspect, a method is provided for predicting stroke risk with an artificial intelligence-based medical system. Values of parameters representing a geometrical shape of a carotid artery of a patient are acquired. Flow information by location is generated within the geometrical shape. The flow information is generated as an output of an artificial intelligence in response to input of the values of the parameters to the artificial intelligence. An image processor predicts the stroke risk from the flow information.

In one implementation, the values are acquired by segmenting the geometrical shape from a medical image. Any parameterization of the geometrical shape may be used, such as acquiring the values of radius and shape (e.g., contour or curvature) for different locations in a slice relative to a center point of the geometrical shape in the slice for different slices along the geometrical shape.

In another implementation, the flow information is generated as wall shear stress for the locations. The locations are distributed throughout the geometrical shape. In another example, the flow information is velocity, pressure, wall shear stress, shear rate, vorticity, and/or helicity for the locations.

As one implementation, the artificial intelligence is a machine-learned recurrent neural network or transformer.

According to an implementation, the artificial intelligence is a machine-learned model trained with synthetically generated carotid models with ground truths from computational fluid dynamics or a reduced order model.

In yet another approach, an uncertainty is assigned for at least one of the parameters. Different possible flows are generated by the artificial intelligence by perturbation of the values of the at least one parameter as input to the artificial intelligence. The possible flow is selected as the flow information based on a comparison of the different possible flows to a measurement of flow from medical imaging. Another approach includes generating the flow information from candidate blood flows generated by the artificial intelligence in response to input of perturbations of the values and selection of the flow information as the candidate blood flow matching a measurement of flow.

According to another implementation, the flow information is wall shear stress. The stroke risk is predicted as an integral of time averaged wall shear stress for a region of the carotid artery.

In a second aspect, a method is provided for stroke risk prediction in a medical system. First values of parameters representing a geometrical shape of a carotid artery of a patient are acquired. Uncertainty is determined for at least one of the parameters. A processor creates second values of the parameters from the first values based on the uncertainty. A machine-learned model generates first and second candidate blood flows as output by the machine-learned model in response to input of the first and second values to the machine-learned model, respectively. The processor selects one of the first and second candidate blood flows based on comparison to a measured flow. The processor predicts the stroke risk for the patient from the selected one of the candidate blood flows.

As an implementation, the geometrical shape is acquired from a medical image by segmentation.

In one implementation, the uncertainty is determined from a relationship of a voxel size of the medical image to radius of the geometrical shape.

According to another implementation, the first values are perturbed to the second values as a random sampling in a range set by the uncertainty.

In another implementation, the uncertainty is determined as a function of location in the geometrical shape. The first values are perturbed to the second values as a function of the location based on the uncertainty as the function of the location.

In yet another implementation, the machine-learned model was trained with synthetically generated carotid models with ground truths from computational fluid dynamics or a reduced order model.

As another implementation, the selected candidate blood flow is used to compute wall shear stress. The stroke risk is predicted as an integral of time averaged wall shear stress for a region of the carotid artery.

In a third aspect, a system is provided for stroke risk prediction. A medical imaging scanner is configured to scan a carotid artery of a patient. An image processor is configured to (a) segment a geometrical model of the carotid artery of the patient from the scan, (b) determine uncertainty of the geometrical model, (c) create perturbed models of the geometrical model from the uncertainty, (d) output, by a machine-learned model, separate candidate flows in response to separate inputs of the geometrical model and the perturbed models to the machine-learned model, (e) select one of the candidate flows based on comparison to a flow measured from the scan, and (f) predict stroke risk for the patient from the selected one of the candidate flows. A display is configured to display the predicated stroke risk.

In an implementation, the machine-learned model is a model machine trained from training data of synthetically generated carotid models and corresponding ground truth flows calculated for the synthetically generated carotid models.

In another implementation, the image processor is configured to determine the uncertainty from a voxel or pixel size for the scan.

These and other aspects, features and advantages will become apparent from the following detailed description of preferred embodiments, which is to be read in connection with the accompanying drawings. The present invention is defined by the following claims, and nothing in this section should be taken as a limitation on those claims. Further aspects and advantages of the invention are discussed below in conjunction with the preferred embodiments and may be later claimed independently or in combination.

### BRIEF DESCRIPTION OF THE DRAWINGS

The components and the figures are not necessarily to scale, emphasis instead being placed upon illustrating the principles of the embodiments. Moreover, in the figures, like reference numerals designate corresponding parts throughout the different views.
Figure 1 is a flow chart diagram of one embodiment of a method for prediction of stroke risk using artificial intelligence;
Figure 2 illustrates example parameterization for generating synthetic carotid geometric models;
Figure 3 illustrates examples of flow information output by an artificial intelligence; and
Figure 4 is a block diagram of one embodiment of a system for stroke risk prediction.

### DETAILED DESCRIPTION OF EMBODIMENTS

Real-time stroke risk is predicted from carotid artery images. Artificial intelligence (AI)-based flow analysis of the carotid bifurcation enables real-time analysis of hemodynamic patterns based on physiological modeling and medical images. A fast processing AI-based model of blood flow is trained on synthetically generated training sets in a multi-step workflow. In inference, uncertainty-driven generation of candidate blood flow scenarios may be used where the final prediction is selected using measurements available in the clinical data, providing increased accuracy in estimated flow.

AI provides real time processing of carotid medical images for estimating subject-specific blood flow features without the need for traditional computational modeling techniques. Fast (i.e., 2 seconds or less and faster than computational fluid dynamics) blood flow modeling enables the generation of multiple scenarios. Each scenario is a set of geometric and hemodynamics features representing the blood flow in the patient-specific geometry reconstructed from medical images. Comparison between the generated multiple scenarios and direct measurements performed using medical imaging or another source of blood flow allows selection of one scenario out of the multiple scenarios. The blood flow features most consistent with the available data are selected. This accurately modeled blood flow scenario is used to assess disturbed blood flow and the associated stroke risk.

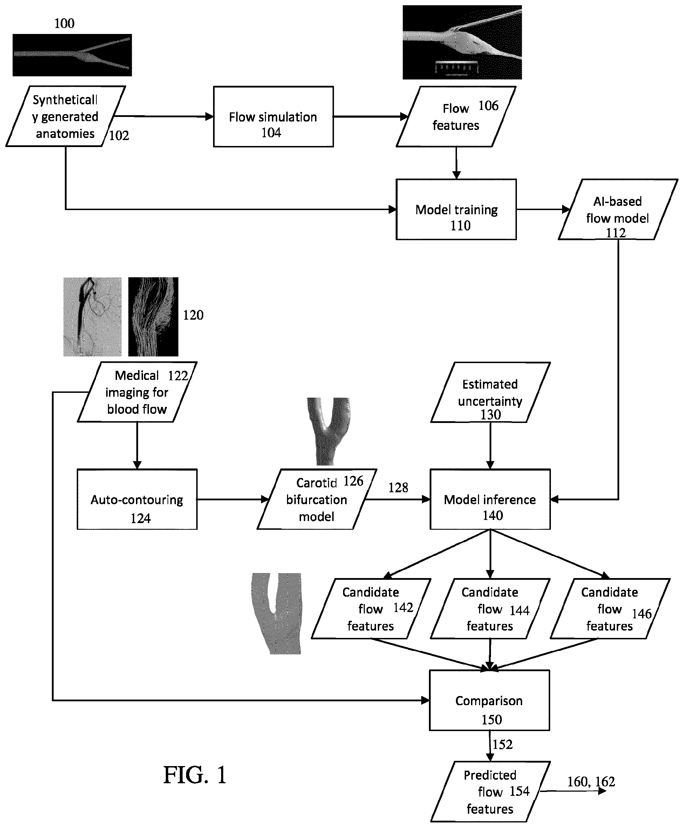

Figure 1 is a flow chart diagram of one embodiment of a method for stroke risk prediction in a medical system. The method is for predicting stroke risk with an AI-based medical system. The AI estimates flow distribution in the carotid in response to input of geometrical shape information of the patient's carotid. In a further implementation, this AI-based estimation may be used to estimate different flows rapidly, so the geometrical shape information may be altered or sampled based on uncertainty of the shape. The different flow distributions are compared to measured flow information to select the distribution matching flow in the patient's carotid for more accurate stroke risk predication from the flow distribution.

The method is performed in the order shown (top to bottom, left to right, and/or numerical). Some acts may be performed in parallel or sequentially. Other orders may be used. For example, act 130 is performed prior to act 124.

Additional, different, or fewer acts may be provided. For example, acts 104 and 110 are for training so may be provided without later acts or may not be provided where an already trained AI is used for inference. As another example, use of uncertainty 130 comparison 150, and selection 152 may not be provided in other implementations. In yet another example, an act for determining uncertainty is included.

The method is performed by a medical diagnostic scanner, a workstation, a server, or a computer. Any device with an image processor may be used. In one approach, a server, workstation, or computer performs the training acts. A different image processor, such as in a medical scanner, hospital workstation, or a server, performs the inference and stroke risk prediction. A display device outputs the predicted risk of stroke with or without one or more medical images, such as images showing distributed flow in the carotid of the patient as estimated by the AI. It is understood that when it is referred to a "means or device for" executing an action the "means or device is configured to or implemented to" carry out the action. In particular, the means/device may be a structural embodiment of a method step or process including the action, e. g. a "means for receiving" may correspond to the "step of receiving".

An image processor acquires 128 values of parameters representing a geometrical shape 126 of a carotid artery of a patient. The values may be received from memory, input from a user interface, or transfer over a computer network. The values are provided from any source, such as a physician inputting the values.

In one implementation, the values are acquired by image processing a medical image 120. An image processor segments (contours) 124 the geometrical shape 126 from the medical image 120.

Medical imaging 122 is performed by any medical scanner imaging the carotid of a patient. The imaging 122 may be for flow and/or anatomy. The medical imaging 122, corresponding medical image 120, and/or medical scanner may be ultrasound (e.g., B-mode anatomy scan and/or Doppler flow scan), magnetic resonance imaging (MRI) (e.g., 4D flow MRI), computed tomography, or digital subtraction angiography. Anatomical scans and/or flow scans may show luminal geometry and/or indicate places of plaque, stenosis, and/or thrombus.

The imaging 122 provides image data (images 120) representing the carotid of the patient. The region of the carotid bifurcation of the patient is imaged. Two- or three-dimensional imaging may be used. For example, the imaging generates images representing different planes or slices of the carotid. Some or all the planes may be parallel or substantially parallel (e.g., +/-10% angle). One or more of the planes may not be parallel (e.g., +/-11% or more angle, such as substantially orthogonal or perpendicular). Each location represented in the image corresponds to a pixel or voxel. Alternatively, a single 2D plane extending longitudinally through the carotid is used. In another alternative, a volume scan provides a distribution of measured values over a grid of voxels. The imaging may be for one time or represent the carotid artery over one or more heart cycles.

The image processor contours or segments 124 a structure of the carotid in the images. The segmentation may be in two or three dimensions. The vascular walls of the carotid with or without any plaque, thrombus, or other structure or flow is segmented.

Any contouring algorithm capable of segmentation of the carotid may be utilized. For instance, random walker, pattern matching, thresholding, shape fitting, or other processes are used to segment the vascular walls. As another example, algorithms based on convolutional neural networks or other machine-learned models for image segmentation are used. Any machine-learned model resulting from machine training may be used. For example, a support vector machine, clustering, image-to-image deep learned neural network, or another generator is trained to generate the segmentation given an input image. In one embodiment, a neural network, such as a U-Net, image-to-image, encoder-decoder, or generator, is trained to generate the segmentation. The neural network may be a fully connected network and/or a convolutional neural network (CNN).

In one implementation, the image processing sequence includes automatic contouring 124 of the carotid bifurcation with segmentation techniques such as disclosed in U. S. Patent 10,762,637. For example, different contours along a center line are extracted. The adjacent contours may be used to limit, initialize, and/or optimize extraction of each of the contours. The resulting carotid bifurcation model 126 represents the three-dimensional shape of the vessel wall in the region of interest surrounding the bifurcation as a set of aligned contours.

The contouring or segmentation 124 provides the geometric model 126. Where contours in different slices are found, the contours may be used as the model or may be combined and/or meshed to form the geometric model 126. Where a shape model is fit to the image, the fit shape model is the geometric model 126. Where an outline or surface is found by segmentation, the outline or surface is used to form a mesh or is the mesh.

Other representations of the geometric model 126 may be used, such as parameterizing the segmented or contoured information without meshing. For example, for each sampling along a center line, radii to a number of different points on the carotid and the curvature at those points of the carotid is determined as the geometric model 126. For example, the image processor acquires 128 the values of radius and shape for different locations in a slice relative to a center point of the geometrical shape in the slice for each of different slices along the geometrical shape. Other parameterizations may be used.

The values of the parameters are acquired 128 as the geometric model 126 or extracted from the geometric model 126. The geometric model 126 may be used as input to the AI 112. Alternatively, information is extracted from the geometric model, such as parameterizing the 3D shape into radii and contour (curvature) for different slices along a center line, and this information is input to the AI 112.

The image processor generates 140 flow information by location within the geometrical shape 126. Flow is determined for each voxel, each sample location for the parameters, along the vascular walls, along the center line, or on a grid of any size. Flow is generated for various locations distributed through at least part of the geometrical shape 126. The flow information may alternatively be for one location, a region, or the entire carotid.

The flow information represents an aspect of the moving fluid (e.g., blood), such as any hemodynamic parameter. For example, the flow information is velocity, acceleration, pressure, wall shear stress, shear rate, vorticity, and/or helicity for one or more locations (e.g., for a distribution of locations). One type of flow information is generated. In other embodiments, two or more types of flow information are generated for each location or for the carotid, such as velocity and wall shear stress or velocity and pressure.

The AI (i.e., AI-based flow model 112) generates the flow information. The flow information is output by the AI 112 as part of inference 140 from the values of the parameters. The values are input to the AI 112, which infers the flow distribution as the output. The trained AI model 112 of blood flow takes the carotid bifurcation model 126 as an input and returns a set of blood flow features 142, 144, 146. In one implementation, only one flow feature (e.g., wall shear stress) 142 is generated. In other implementations, values for multiple features 142, 144, 146 are generated, such as blood velocity and blood pressure or another combination of features 142, 144, 146 indicating complex patterns of flow. The AI model 112 returns the value of the set of blood features 142, 144, 146 evaluated at each of a multitude of points within the carotid bifurcation model 126.

The AI 112 is a machine-learned model. Any machine learning and corresponding architecture may be used. For example, a neural network is used, such as convolutional or fully connected neural network. A U-net, image-to-image, encoder-decoder, generator, or another network may be used. In one implementation, the machine-learned model is a recurrent neural network or transformer that relates the values for different locations as a sequence of geometric features organized along the centerline of the vessel to estimate the flow for the distribution of locations. In another implementation, the AI-based model 112 of blood flow is trained as disclosed in U.S. Published Patent Application No. 2021/0219935 or U.S. Patent No. 11,589,924, such as a transformer using thresholding.

The machine-learned model is trained 110 with training data. Many (e.g., hundreds, thousands, or more) samples of inputs and ground truth output are used to machine train. A training sample 102 includes a pair of two objects: a 3D geometry representing the carotid bifurcation and a 3D field of values representing blood flow features in each space location within the 3D geometry.

The training data is collected from medical records. Alternatively, or additionally, the training data includes synthetically created samples. Some situations may be uncommon so under-represented in the collection of training data. It may be difficult to collect a large enough collection for accurate machine learning. Synthetic samples may fill the gaps. The training 110 of the AI model 112 of blood flow may rely on synthetically generated training samples 102.

For example, carotid models representing the geometry of the carotid are generated. A shape model, fit mesh, segmented model, or parameterized model for a patient or patients may be altered to create synthetic models. Simulation, physics, or biomechanical modeling may be used to create synthetic models without starting with a patient-based model. The 3D geometry representing a carotid bifurcation may be based on the generation of simple shapes (e.g., tubes with variable radius), parameterized by a set of shape characteristics.

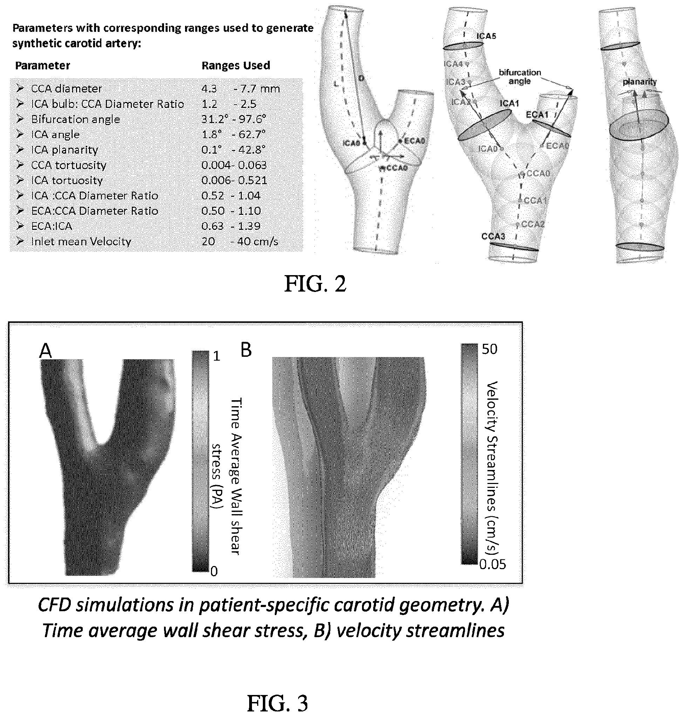

Figure 2 shows an example parameterization used to generate synthetic geometrical models. A list of parameters parameterizing the shape of the carotid bifurcation is depicted in Figure 2 with common carotid, internal carotid, and external carotid artery (CCA, ICA, and ECA) parameters. Generation of multiple instances of the carotid geometries is achieved by randomly sampling each parameter from a statistical distribution (e.g., uniform distribution) within a given range of variability. The different parameter values that correspond to the ranges of patient-specific geometries may be provided by literature or studies. The parameterization used for creating the synthetic samples is the same or different than the parameterization input to the machine-learned model 112.

The ground truth is generated by calculation or modeling. For example, computational fluid dynamics is used to determine the flow (hemodynamics) for each of the synthetic carotid models. Generation of a 3D field of values representing blood flow in a given 3D geometry is obtained by the solution of a computational fluid dynamics problem. In one implementation, 3D Navier-Stokes equations are solved by a computational model to obtain velocity and pressure values of blood at each location in the 3D geometry. Figure 3 shows a visual example of values representing the blood flow in two dimensions in a 3D geometry of the carotid bifurcation. The calculated ground truths in this example are time average wall shear stress (A) and velocity (B). The velocity is represented as streamlines. A vector field may be used instead.

In another implementation, a reduced order model of blood flow is used to calculate the ground truth. Spatially averaged estimates of blood flow features, such as flow rate or average pressure, on axial cross-sections of the synthetic geometrical model are estimated by the reduced order model as the ground truth.

The training 110 uses the samples. Values of learnable parameters of the AI architecture are learned through optimizing to minimize a difference between inferred output flow and ground truth flow given the input. Once trained, the AI-based flow model 112 may be used for inference 140 based on input 128 of previously unseen values of parameters for a geometrical model 126.

In one implementation, the AI-based flow model 112 uses uncertainty 130 for the inference 140 of flow from the values of the parameters of the geometric model 126 of the patient's carotid. The uncertainty 130 is for accuracy of the image 120, inference 140, segmentation 124, the geometrical model 126, and/or other processes or systems. The reliability of the data is known and/or measured to represent the uncertainty.

For example, a given voxel in the image 120 or location of the parameterization of the model 126 may represent both flow and structure due to the area or volume covered. Uncertainty 130 may derive from limited spatial or temporal resolution of the original medical images, as well as image artifacts or poor image quality. The ratio of that area or volume to the size of the carotid at that location may represent an uncertainty 130 in accuracy of delineation of the carotid or flow. Such uncertainty 130 can be associated to the precise definition of the geometry model, in particular the position in space of the vascular wall. The uncertainty 130 is determined from the relationship of voxel or pixel size of the medical image 120 to radius of the geometric model 126 in one implementation. The uncertainty 130 is used in the inference 140 or application of the inference 140. Uncertainty 130 of the estimated vessel wall position in space is estimated from the spatial resolution of the medical image. Given that the pixel size of the image is X, the uncertainty, Y, in the position of the vessel wall is a multiple of X. A look-up table or function may relate the relationship of radius to pixel or voxel size to uncertainty or relate the voxel or pixel size without relation to the radius to uncertainty.

Based on the estimated uncertainty 130 in the original data, the geometry features 128 of the carotid bifurcation are perturbed, forming other sets of values of the parameters. Based on this estimated uncertainty 130, the geometrical features of the carotid bifurcation, which depend on the vessel position are perturbed to generate multiple inputs for the AI blood flow model. The uncertainty defines a uniform range and/or distribution around the initial value for the geometric parameters (e.g., radius) for random or regular sampling to form other inputs. One example of such geometry features is the local radius of the carotid artery, but the curvature or other geometric parameters (features) may be perturbed based on uncertainty 130. The local radius of the carotid artery may be defined as the distance between each point on the vessel wall from the vessel centerline. If radius R represents the local radius in the original carotid bifurcation model and knowing that the uncertainty 130 on the location of the vessel wall is Y, multiple variations of the local radius can be produced by randomly sampling a distribution of values (e.g., uniform distribution) in the range [R-Y, R+Y]. Each set is provided as input to the AI-based blood flow model 112.

Other measures of uncertainty 130 may be used. Uncertainty may be applied in different ways, such as a weight multiplying the value to perturb.

The uncertainty 130 is determined location-by-location. Different uncertainties 130 are determined by the image processor for different locations relative to the geometric model 126. Alternatively, uncertainty 130 is determined as one value (e.g., average) for a region (e.g., slice). A global uncertainty 130 may be used instead or in addition to location or local uncertainty 130. The uncertainty is the same or different over time (i.e., for different parts of a heart cycle).

The AI-based flow model 112 may receive the measurement of uncertainty 130 as an input for estimating the flow at that and/or other locations. Alternatively, the uncertainty 130 is used for the process applying the AI-based flow model 112. The uncertainty 130 defines a tolerance, range, or distribution for one or more parameters input to the AI-based flow model 112. The value for that parameter or values for different parameters may be perturbed or altered by the image processor based on the uncertainty or sampling over a range based on the uncertainty. One set of values are perturbed or altered to create another set of input values of the parametric model 126. The AI-based flow model 112 is used to estimate flows (e.g., candidate flow features 142, 144, 146) for different sets of values, such as two, three, or more.

The resulting estimated candidate flow features 142, 144, 146 may be reviewed or compared to select a better one. The uncertainty is used to create different possible flow estimates to find a better or more likely flow estimate. The uncertainty is used to generate candidate blood flows generated by the AI in response to input of perturbations of the values. The flow information for the patient's carotid is selected as the candidate blood flow matching a measurement of flow or another criterion.

In one implementation, the trained AI model 112 produces multiple candidates of blood flow patterns (e.g., candidates 142, 144, 146, ...) in the carotid bifurcation. Given the 3D geometry of the carotid bifurcation, the AI model 112 is trained to produce the set of blood flow features 142. Alternative blood flow features 144, 146 are produced when accounting for uncertainty in the data acquisition and data processing. Each instance in the set of models 126 of carotid bifurcation for which selected geometry features have been varied according to the estimated uncertainty 130 in the data is then provided as input 128 to the AI blood flow model 112. This produces an equal number of candidate blood flow scenarios 142, 144, 146, from which blood flow features can be computed.

The image processor selects 152 one of the candidate blood flow features 142, 144, 146 (one of the AI estimated flow distributions). The selection is based on a comparison 150. One of the possible flows 142, 144, 146 is selected as the flow information to use for prediction of stroke risk.

The comparison 150 may be to a threshold, expected flow, measurement of flow, or another source of flow information. The estimated flow features 142, 144, 146 are compared to find the expected or more likely flow. For example, the different possible flows 142, 144, 146 as estimated are compared to a measurement of flow from medical imaging. The computed blood flow features 142, 144, 146 are compared with one or more direct measurements performed for or represented in the medical images of blood flow. One example of blood flow features that can be directly compared against direct measurements is maximal velocity in the carotid bifurcation. The blood flow scenario 142 featuring the estimated blood flow velocity field for which the maximal velocity value is the closest to the one obtained from direct measurement in the medical image is selected as the one predicted blood flow scenario 154. Flow from different locations may be averaged, and the averages compared. A minimum sum of absolute differences in a region of interest or the entire carotid bifurcation region from the expected or measured flow may be used to compare 150.

The estimated blood flow 142, 144, 146 may include estimates of flow that are measured by imaging and flow that is not measured. For example, the velocity and wall shear stress are estimated by the machine-learned model 112 in the inference 140. The medical images 120 may include point-wise velocities or flow field of velocity. The estimated and measured velocites are compared 150 to select the estimated or candidate flow 142, 144, 146 most similar to the measured velocities. The corresponding wall shear stress for the candidate flow 142, 144, 146, with or without the selected velocity for the candidate flow 142, 144, 146 is used in the prediction of stroke risk. In another example, the blood pressure in the carotid bifurcation is measured using or not using medical imaging. The machine-learned blood flow model 112 may output pressure for the carotid or pressure as a function of location. These estimated pressures are compared 150 to the measured pressure to select the candidate flow 142, 144, 146 with the most similar pressure.

Additional blood flow features can be derived, such as blood pressure and shear stress values. The estimated flow 142 selected may be used for further calculations and/or analysis to generate information used for stroke prediction. Alternatively, or in addition, the estimated flow field 142 itself is used for the prediction.

The image processor predicts 160 the stroke risk from the selected flow information 142. From the blood flow features 142 produced by the model 112, risk predictors for atherosclerosis disease progression or risk of stroke are derived. An example of a risk predictor based on flow features is a function of wall shear stress values in a region of interest of the carotid bifurcation. Given the integral of time averaged wall shear stress *TA WSS computed* on the vessel wall of the carotid artery, a simple risk predictor may be defined as the function r *= f*(*TAWSS*) = k *TAWSS,* where k is a numeric positive constant, so that the risk for disease progression or stroke events increases with increasing values of integral time averaged wall shear stress. In another example, a weighted combination of vorticity and helicity for a region adjacent to the vascular wall and/or another region of the carotid is used to predict stroke risk. Any function or study-based look up table relating flow to stroke risk is implemented by the processor to predict stroke risk using, at least in part, the selected flow information 142. Other variables or factors may be used, such as clinical information (e.g., history of strokes, family history, blood work measurements, medications, and/or blood pressure), in addition to the flow information estimated by or derived from an estimate by the AI-based blood flow model 112.

In a further act, the predicted stroke risk is output 162. The output 162 may be to a display, such as providing a color coding, alphanumerical text, graph, highlighting, period, or other information indicating the risk of stroke. Other outputs may be provided, such as outputting the estimated flow field 142 as an image with the predicated stroke risk.

The image processor, using a display, displays the selected flow information and/or the predicted stroke risk. Other information may additionally or alternatively be displayed. For example, the original medical images 120 are displayed. Other patient information may be displayed. A quantity or quantities for the geometrical model 126 of the selected candidate flow 142 and/or for the candidate flow 142 may be displayed. A rendering of the geometrical model 126 may be displayed.

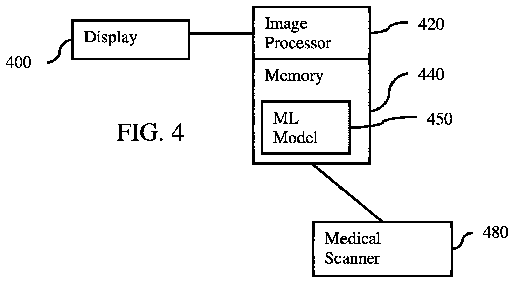

Figure 4 shows one embodiment of a medical system or imager for stroke risk prediction. The system uses AI to estimate flow from input values of parameters for a shape of the carotid, such as at the bifurcation. The system may apply the AI multiple times based on uncertainty driven candidates of the shape and select the resulting estimated flow by comparison to another measure. The system may implement the method of Figure 1.

The medical system includes the display 400, memory 440, and image processor 420. A medical scanner 480 may be included in the medical imager or system. The display 400, image processor 420, and memory 440 may be part of the medical scanner 480, a computer, server, workstation, or other system for image processing medical images from a scan of a patient or processing parameterization of segmented vascular structure. A workstation or computer without the medical scanner 480 may be used as the medical system or imager.

Additional, different, or fewer components may be provided. For example, a computer network is included for remote image processing or data storage based on locally captured scan and/or other imaging data. As another example, a user input device (e.g., keyboard, buttons, sliders, dials, trackball, mouse, or other device) is provided for user input.

The medical scanner 480 is a computed tomography, magnetic resonance, ultrasound, fluoroscopy, angiography (e.g., digital subtraction angiography), x-ray, optical coherence tomography, intracardiac imaging, or another mode of scanner. For example, the medical scanner 480 is a computed tomography system having an x-ray source and detector connected to a moveable gantry on opposite sides of a patient bed. As another example, the medical scanner 480 is a cardiac MRI scanner using a main magnet, gradient coils, local coils, and/or body coil for 4D flow MRI.

The medical scanner 480 is configured by settings to scan the carotid a patient. The scan samples in the patient along 2D planes or a volume over at least one heartbeat or for one time. The scan results in scan or image data that may be processed to generate images of the interior of the patient on the display 400.

The image processor 420 is a control processor, general processor, digital signal processor, three-dimensional data processor, graphics processing unit, application specific integrated circuit, field programmable gate array, artificial intelligence processor or accelerator, digital circuit, analog circuit, combinations thereof, or other now known or later developed device for processing medical image data. The image processor 420 is a single device, a plurality of devices, or a network. For more than one device, parallel or sequential division of processing may be used. Different devices making up the image processor 420 may perform different functions. In one embodiment, the image processor 420 is a control processor or other processor of a medical diagnostic imaging system, such as the medical scanner 480. The image processor 420 operates pursuant to stored instructions, hardware, and/or firmware to perform various acts described herein.

In one implementation, the image processor 420 is configured to (a) segment a geometrical model of the carotid artery of the patient from the scan, (b) determine uncertainty of the geometrical model, (c) create perturbed models of the geometrical model from the uncertainty, (d) output, by a machine-learned model 450, separate candidate flows in response to separate inputs of the geometrical model and the perturbed models to the machine-learned model 450, (e) select one of the candidate flows based on comparison to a flow measured from the scan, and (f) predict stroke risk for the patient from the selected one of the candidate flows. The uncertainty may be determined from a voxel or pixel size for the scan, such as a relative size of the voxel or pixel to a radius of the carotid. In other implementations, uncertainty is not used, such as where parameterization is used to input to the machine-learned model 450 for estimating flow alone (i.e., perform (a), (d) for one flow, and (f)).

The machine-learned model 450 is a model machine trained from training data to estimate flow from carotid structure, such as reflected in values of parameters of the carotid structure. In one implementation, the machine-learned model 450 was trained with training data of synthetically generated carotid models and corresponding ground truth flows calculated for the synthetically generated carotid models.

The image processor 420 is configured to generate an image. The image shows the selected geometric model, a quantity derived from the selected geometric model, an output estimate of flow and/or information derived from the estimated flow, the uncertainty, and/or the predicted stroke risk.

The display 400 is a CRT, LCD, projector, plasma, printer, tablet, smart phone, or other now known or later developed display device for displaying the output, such as an image with the predicted stroke risk.

The training data, machine-learned model 450, image data, non-image data, segmentation, geometric model, uncertainty, candidate flows, measurement, predicated stroke risk, and/or other information are stored in a non-transitory computer readable memory, such as the memory 440. The memory 440 is an external storage device, RAM, ROM, database, and/or a local memory (e.g., solid state drive or hard drive). The same or different non-transitory computer readable media may be used for the instructions and other data. The memory 440 may be implemented using a database management system (DBMS) and residing on a memory, such as a hard disk, RAM, or removable media. Alternatively, the memory 440 is internal to the processor 420 (e.g., cache).

The instructions for implementing, by execution by the processor 420, the acts, the methods, and/or the techniques discussed herein are provided on non-transitory computer-readable storage media or memories, such as a cache, buffer, RAM, removable media, hard drive, or other computer readable storage media (e.g., the memory 440). Computer readable storage media include various types of volatile and nonvolatile storage media. The functions, acts or tasks illustrated in the figures or described herein are executed in response to one or more sets of instructions stored in or on computer readable storage media. The functions, acts or tasks are independent of the particular type of instructions set, storage media, processor or processing strategy and may be performed by software, hardware, integrated circuits, firmware, micro code and the like, operating alone or in combination.

In one embodiment, the instructions are stored on a removable media device for reading by local or remote systems. In other embodiments, the instructions are stored in a remote location for transfer through a computer network. In yet other embodiments, the instructions are stored within a given computer, CPU, GPU, or system. Because some of the constituent system components and method steps depicted in the accompanying figures may be implemented in software, the actual connections between the system components (or the process steps) may differ depending upon the way the present embodiments are programmed.

Various improvements described herein may be used together or separately. Although illustrative embodiments of the present invention have been described herein with reference to the accompanying drawings, it is to be understood that the invention is not limited to those precise embodiments, and that various other changes and modifications may be affected therein by one skilled in the art without departing from the scope of the invention.

## Claims

1. A method for predicting (140) stroke risk with an artificial intelligence-based medical system, the method comprising:
acquiring (100) values of parameters representing a geometrical shape of a carotid artery of a patient;
generating (140) flow information by location within the geometrical shape, the flow information generated as an output of an artificial intelligence in response to input of the values of the parameters to the artificial intelligence; and
predicting (152), by an image processor (420), the stroke risk from the flow information.

2. The method of claim 1, wherein acquiring (100) comprises segmenting the geometrical shape from a medical image.

3. The method of claim 1 or 2, wherein acquiring (100) comprises acquiring (100) the values of radius and shape for different locations in a slice relative to a center point of the geometrical shape in the slice for different slices along the geometrical shape.

4. The method of any one of claims 1-3, wherein generating (140) the flow information comprises generating (140) wall shear stress for the locations, the locations distributed throughout the geometrical shape.

5. The method of any one of claims 1-4, wherein generating (140) the flow information comprises generating (140) velocity, pressure, wall shear stress, shear rate, vorticity, and/or helicity for the locations, the locations distributed throughout the geometrical shape.

6. The method of any one of claims 1-5, wherein generating (140) comprises generating (140) by the artificial intelligence comprising a machine-learned recurrent neural network or transformer.

7. The method of any one of claims 1-6, wherein generating (140) comprises generating (140) by the artificial intelligence comprising a machine-learned model (450) trained with synthetically generated carotid models with ground truths from computational fluid dynamics or a reduced order model.

8. The method of any one of claims 1-7, wherein generating (140) comprises assigning an uncertainty for at least one of the parameters, generating (140) different possible flows by the artificial intelligence by perturbation of the values of the at least one parameter as input to the artificial intelligence, and selecting the possible flow as the flow information based on a comparison of the different possible flows to a measurement of flow from medical imaging.

9. The method of any one of claims 1-8, wherein generating (140) comprises generating (140) the flow information from candidate blood flows generated by the artificial intelligence in response to input of perturbations of the values and selection of the flow information as the candidate blood flow matching a measurement of flow.

10. The method of any one of claims 1-9, wherein the flow information comprises wall shear stress, and wherein predicting (140) comprises predicating the stroke risk as an integral of time averaged wall shear stress for a region of the carotid artery.

11. A method for stroke risk prediction in a medical system, the method comprising:
acquiring (100) first values of parameters representing a geometrical shape of a carotid artery of a patient;
determining (130) uncertainty for at least one of the parameters;
creating (128), by a processor (420), second values of the parameters, the second values generated from the first values based on the uncertainty;
generating (140), by a machine-learned model (450), first and second candidate blood flows as output by the machine-learned model (450) in response to input of the first and second values to the machine-learned model (450), respectively;
selecting (150), by the processor (420), one of the first and second candidate blood flows based on comparison to a measured flow; and
predicting (152), by the processor (420), the stroke risk for the patient from the selected one of the candidate blood flows.

12. The method of claim 11, wherein acquiring (100) comprises segmenting the geometrical shape from a medical image.

13. The method of claim 11 or 12, wherein determining (130) comprises determining the uncertainty from a relationship of a voxel size of the medical image to radius of the geometrical shape.

14. The method of any one of claims 11 - 13, wherein creating (128) comprises perturbing the first values to the second values as a random sampling in a range set by the uncertainty.

15. The method of any one of claims 11 - 14, wherein determining (130) comprises determining the uncertainty as a function of location in the geometrical shape, and wherein creating (128) comprises perturbing the first values to the second values as a function of the location based on the uncertainty as the function of the location.

16. The method of any one of claims 11 - 15, wherein generating (140) comprises generating (140) by the machine-learned model (450) having been trained with synthetically generated carotid models with ground truths from computational fluid dynamics or a reduced order model.

17. The method of any one of claims 11 - 16, wherein the selected candidate blood flow is used to compute wall shear stress, and wherein predicting (140) comprises predicting (140) the stroke risk as an integral of time averaged wall shear stress for a region of the carotid artery.

18. A system for stroke risk prediction, the system comprising:
a medical imaging scanner (480) configured to scan a carotid artery of a patient;
an image processor (420) configured to (a) segment a geometrical model of the carotid artery of the patient from the scan, (b) determine uncertainty of the geometrical model, (c) create perturbed models of the geometrical model from the uncertainty, (d) output, by a machine-learned model (450), separate candidate flows in response to separate inputs of the geometrical model and the perturbed models to the machine-learned model (450), (e) select one of the candidate flows based on comparison to a flow measured from the scan, and (f) predict stroke risk for the patient from the selected one of the candidate flows; and
a display (400) configured to display the predicated stroke risk.

19. The system of claim 18, wherein the machine-learned model (450) comprises a model machine trained from training data of synthetically generated carotid models and corresponding ground truth flows calculated for the synthetical generated carotid models.

20. The system of claim 18 or 19, wherein the image processor (420) is configured to determine the uncertainty from a voxel or pixel size for the scan.
